# EUROPÄISCHE PATENTSCHRIFT

(11) **EP 0 599 847 B1**
(45) Veröffentlichungstag und Bekanntmachung des Hinweises auf die Patenterteilung: **02.04.1997**
(21) Anmeldenummer: 92911746.3
(22) Anmeldetag: 25.06.1992
(51) Int. Cl.: A61B 17/60

(54) **OSTEOSYNTHETISCHE FIXATIONSVORRICHTUNG**
OSTEOSYNTHETIC FIXATION DEVICE
DISPOSITIF DE CONTENTION OSTEOSYNTHETIQUE

(43) Veröffentlichungstag der Anmeldung: 08.06.1994
(73) Patentinhaber: Synthes AG, Chur, CH-7002 Chur (CH)
(72) Erfinder: SCHLÄPFER, Johannes, Fridolin, CH-8750 Glarus (CH); HESS, Martin, CH-4434 Hölstein (CH)
(74) Vertreter: Lusuardi, Werther Giovanni, Dr.
(86) Internationale Anmeldenummer: CH9200125
(87) Internationale Veröffentlichungsnummer: WO9400066

(56) Entgegenhaltungen:
- EP-A- 0 216 563
- EP-A- 0 450 075
- WO-A-88/03781
- DE-A- 3 027 138
- DE-A- 3 027 148
- DE-U- 8 609 102
- FR-A- 2 640 493

## Beschreibung

Die Erfindung bezieht sich auf eine osteosynthetische Fixationsvorrichtung gemäss der Gattung des Patentanspruchs 1.

In der Osteosynthese ergeben sich vielfältige Bedürfnisse nach gegenseitiger Fixation der involvierten Knochenfragmente. Es sind deshalb bereits eine grosse Zahl von Fixationsvorrichtungen bekannt, beispielsweise Platten/Schrauben-Systeme, Fixateurs externes, Fixateurs internes, Wirbelsäulenfixationssysteme u.s.w.
Viele dieser bekannten Vorrichtungen erlauben lediglich ein Arbeiten in zwei Dimensionen, was deren Anwendbarkeit stark einschränkt. Ein weiterer Nachteil liegt in der fehlenden oder nur in geringem Masse vorhandenen Möglichkeit die Fixationsvorrichtung intraoperativ zu adaptieren, d.h. die Verbindung zwischen den einzelnen Fixationselementen rasch und einfach wieder zu lösen und erneut in einer anderen relativen Stellung zueinander zu blockieren.

Aus der DE-A- 30.27.148 ist beispielsweise eine Knochenplatte mit einem nach oben halbkugelförmig sich öffnenden Schraubenloch bekannt, in welches ein kugeliges, geschlitzes Klemmelement mit konischer Bohrung eingelegt werden kann. Durch die konische Bohrung des in der Platte gelagerten Klemmelementes hindurch kann eine Knochenschraube mit entsprechendem konischem Kopf in den Knochen eingedreht werden bis der konische Schraubenkopf in der konischen Innenbohrung des Klemmelement zur Anlage kommt, dieses aufweitet und innerhalb des Schraubenlochs der Platte verklemmt.
Der Nachteil bei dieser bekannten Vorrichtung liegt im Umstand begründet, dass die Knochenschraube nicht rigide mit der Knochenplatte verbunden ist. Sobald die Schraube nicht mehr fest im Knochenmaterial verankert ist, kann sie sich leicht vom Klemmelement oder zusammen mit diesem von der Platte lösen. Das offenbarte Verankerungsprinzip kann deshalb nur auf Knochenplatten angewendet werden. Die Übertragung des Prinzips auf einen Fixateur externe oder ein Wirbelsäulenfixationssystem ist ausgeschlossen, da es an einer rigiden Verbindung zwischen den einzelnen Elementen fehlt; die Fixation wird temporär nur solange aufrechterhalten als die Knochenschraube fest im Knochenmaterial sitzt und die Knochenplatte gegen den Knochen drückt.

Aus der EP-A2 355 035 ist weiter eine Knochenplatte bekannt, bei der das Schraubenloch analog zur DE-A- 30.27.148 mit einer kugelzonenförmigen Innenwandung versehen ist, welche sich aber hier beidseits eines Grosskreises erstreckt, d.h. gegen beide Plattenseiten hin verengt. Im weiteren ist hier auch eine Selbsthemmung der Bauelemente offenbart. Nachteilig bei dieser bekannten Vorrichtung sind jedoch die folgenden Umstände:
- Das Verklemmen der Schraube im Klemmelement erfolgt rotativ, gleichzeitig mit ihrem Eindrehen in den Knochen mit Hilfe eines Sechskantschlüssels. Dabei treten rasch anwachsende Reibungskräfte zwischen den konischen Anlageflächen der beiden Elemente auf, was das Eindrehen der Knochenschraube behindert.
- Die Schraube kann nur nach erfolgter Positionierung der Knochenplatte im Knochen fixiert werden. Eine Befestigung der bereits implantierten Schraube an der Platte, bzw. einem anderen als Verbindungselement wirkenden Teil ist nicht möglich.
- Das Prinzip funktioniert nur, solange die Platte fest am Knochen aufliegt und der Knochen gute Verankerungseigenschaften aufweist. Sobald die Platte nicht mehr auf dem Knochen aufliegt, funktioniert die Verbindung nicht mehr.

Schliesslich ist aus der WO 88/03781 eine osteosynthetische Vorrichtung bekannt, welche aus einer Knochenplatte und mehreren darin befestigbaren Knochenschrauben als Fixationselemente besteht, wobei die kugelförmigen Köpfe der Knochenschrauben mittels einer konischen Einsatzschraube spreizbar ausgebildet sind, so dass eine starre Verbindung zwischen Schraubenkopf und Knochenplatte realisiert werden kann. Der Gewindeschaft der Einsatzschraube kann hier als axial am Fixationselement angeordnetes Zugelement betrachtet werden. Das axial angeordnete Zugelement ist auch bei dieser bekannten Vorrichtung, gleich wie bei derjenigen gemäss der DE-A- 30.27.148, in Richtung der Längsachse des Fixationselementes gesehen, auf der selben Seite des konischen Kopfteils, d.h. der Einsatzschraube, angeordnet wie das Verankerungsteil.

Hier will die Erfindung Abhilfe schaffen. Der Erfindung liegt die Aufgabe zugrunde, eine dreidimensional adaptierbare, osteosynthetische Fixationsvorrichtung zu schaffen, welche eine rasch und einfach blockierbare und deblockierbare, rigide Fixation der einzelnen Elemente zueinander gestattet.

Die Erfindung löst die gestellte Aufgabe mit einer osteosynthetischen Fixationsvorrichtung, welche die Merkmale des Anspruchs 1 aufweist.

Weitere vorteilhafte Ausgestaltungen der Erfindung sind in den abhängigen Ansprüchen gekennzeichnet.

Nachstehend wird nun das allgemeine Klemmprinzip der erfindungsgemässen Fixationsvorrichtung beschrieben.
Je nach Ausführungsform der Erfindung wird das eigentliche im oder am Knochen zu verankernde Fixationselement in die konische Bohrung des kugelschichtförmigen, im Verbindungselement gelagerten Klemmelementes eingefahren oder letzteres wird über den konischen Kopfteil des Fixationselementes geschoben. Das Verbindungselement kann seinerseits mit einem Längsträger verbunden sein. Anschliessend wird ein geeignetes Instrument benützt um den Winkel zwischen dem Verbindungselement, bzw. dem darin eingeführten Längsträger und dem Fixationselement einzustellen (z.B. Reposition einer Wirbelfraktur) und gleichzeitig den Konus zu blockieren. Der erwähnte Winkel kann in allen Richtungen, d.h. dreidimensional angepasst werden, indem das Instrument wie ein Steuerknüppel hin- und hergeschoben wird. Durch ein Drehen des Instrumentes im Uhrzeigersinn wird das Fixationselement immer mehr in das Klemmelement hineingeschoben. Dadurch wird letzteres gespreizt und blockiert unter gleichzeitiger Verklemmung des Konus.
Das Instrument wirkt bei diesem Vorgang somit als Mutter, in welche das als Spindel wirkende Zugelement der Fixationsvorrichtung eingeschraubt und damit relativ zur Längsachse verschoben wird. Die Verkeilung des Fixationselementes im Klemmelement erfolgt folglich nicht durch eine rotative Bewegung sondern allein durch eine axiale Verschiebung der beiden Elemente gegeneinander.

Bei einer bevorzugten Ausführungsform ist der Konus selbsthemmend ausgebildet, dadurch dass der Konuswinkel des konischen Kopfteils und der konischen Bohrung im Klemmteil etwa 4° beträgt. Die Selbsthemmung hat den Vorteil, dass die hergestellte Verbindung zwischen den Bauelementen nach Entfernung des Instrumentes nicht wieder auseinanderfällt. Je nach Ausführungsform kann entweder eine Mutter oder eine Schraubkappe mit Innengewinde zur Sicherung verwendet werden. Die Mutter, bzw. Schraubkappe wird normalerweise nicht dazu benutzt das Fixationselement in die konische Bohrung des Klemmelementes hineinzuziehen, bzw. das Klemmelement über den Konus des Fixationselementes zu ziehen. Bei einem sehr flachen Konuswinkel, kann die Mutter oder Schraubkappe als Sicherungselement auch weggelassen werden.

Das Klemmelement kann entweder fest - aber drehbar - in der kugelschichtförmigen Bohrung des Verbindungselementes gelagert sein oder durch geeignete Ausgestaltung auch entfernbar sein. Zu diesem Zweck wird die kugelschichtförmige Bohrung des Verbindungselementes an einer ihrer beiden Öffnungen mit zwei um 180° versetzt angeordneten Ausnehmungen versehen. Dadurch ist ein Einsetzen und Entfernen des Klemmteils ohne Kraftanwendung möglich, indem letzterer um 90° gedreht und aus seinem Sitz herausgedrückt werden kann.

Vorzugsweise verjüngt sich der konische Kopfteil des Fixationselementes in Richtung seines freien, vom Verankerungsteil abgewandten Endes, da dies die nachträgliche Befestigung am Verbindungselement von unten her erleichtert, bzw. erst gestattet. Es ist jedoch auch ein umgekehrter Konus möglich, der von oben in das Verbindungselement eingeführt werden muss.

Der konische Kopfteil ist zweckmässigerweise einstückig mit dem Verankerungsteil verbunden; er kann jedoch auch als separater Teil, beispielweise als Hohlkegel ausgebildet sein.

Die, die Spreizbarkeit des Klemmelementes bewirkenden, quer zum Grosskreis des Klemmelementes verlaufenden Schlitze sind vorzugsweise alternierend, einmal von oben und einmal von unten angeordnet und mit Vorteil an einer Stelle durchgehend. Eine andere Möglichkeit besteht darin, das Klemmelement nur auf der Seite des grösseren Durchmessers seiner konischen Bohrung mit Schlitzen zu versehen.

Im weiteren hat es sich als zweckmässig erwiesen die kugelzonenförmige Oberfläche des Klemmelementes und/oder die kugelige Innenfläche des Verbindungselementes aufzurauhen, z.B. durch Korundstrahlen oder zu strukturieren, z.B. durch Anbringen einer scharfkantigen Nut in der kugeligen Innenfläche des Verbindungselementes kombiniert mit einem Klemmelement aus einem weicheren Material. Eine andere Variante besteht darin die kugelzonenförmige Oberfläche des Klemmelementes zu strukturieren, z.B. mit scharfen Kanten und mit einem Verbindungselement aus einem relativ weichen Material zu kombinieren.

Das Verbindungselement ist vorzugsweise mit einem kreiszylindrischen Kanal versehen, in welchem ein Längsträger aufgenommen werden kann. Dies gestattet beispielswiese die Anwendung der erfindungsgemässen Vorrichtung im Wirbelsäulenbereich. Für spezielle Anwendungen, z.B. im Sakralbereich kann der Kanal eine Neigung gegenüber der Horizontalebene des hier als Sakralbacke ausgebildeten Verbindungselementes aufweisen, z.B. von 25°.

Das erfindungsgemässe Zugelement ist vorzugsweise als ein axial mit dem Kopfteil des Fixationselementes fluchtender kreiszylindrischer Abschnitt mit Aussengewinde ausgebildet. Es ist jedoch auch möglich das Zugelement als ein axial mit dem Klemmteil fluchtender kreiszylindrischer Abschnitt mit Aussengewinde zu realisieren.

Mit dem erfindungsgemässen Klemmprinzip ist es möglich auch mehrere Fixationselemente, z.B. Knochenschrauben untereinander zu verbinden. Auch die Realisation als einseitiger, doppelseitiger oder verstellbarer Fixateur externe oder interne ist möglich.

Die durch die Erfindung erreichten Vorteile sind im wesentlichen darin zu sehen, dass dank der erfindungsgemässen Fixationsvorrichtung insgesamt eine geringe Bauhöhe erzielt wird, eine dreidimensionale Adaptabilität gewährleistet ist, das Reponieren der Knochenfraktur und Blockieren der Fixationsvorrichtung simultan in einem Zug erfolgt, die Klemmung durch eine reine Translation des Klemmelementes gegenüber dem Fixationselement erzeugt wird und dass durch eine geeignete Materialpaarung von Klemmelement und Fixationselement die zwischen den beiden Elementen auftretende Reibung minimal gehalten wird.

Die Erfindung und Weiterbildungen der Erfindung werden im folgenden anhand der teilweise schematischen Darstellungen mehrerer Ausführungsbeispiele noch näher erläutert.

Es zeigen:
Fig. 1 eine Axialschnittdarstellung der erfindungsgemässen Fixationsvorrichtung;
Fig. 2 eine Perspektivansicht des Klemmteils gemäss Fig. 1;
Fig. 3 eine Ansicht von unten des Verbindungselementes gemäss Fig. 1;
Fig. 4 eine partielle Axialschnittdarstellung einer modifizierten Ausführungsform der Erfindung;
Fig. 5 eine Perspektivansicht einer Knochenplatte mit mehreren Fixations- und Klemmelementen;
Fig. 6 eine Perspektivansicht einer als doppelseitiger Fixateur interne dienender erfindungsgemässen Fixationsvorrichtung;
Fig. 7 eine Perspektivansicht einer als einseitiger Fixateur interne dienender erfindungsgemässen Fixationsvorrichtung;
Fig. 8 eine Perspektivansicht einer als längsverstellbarer Fixateur interne dienender erfindungsgemässen Fixationsvorrichtung;
Fig. 9 eine Axialschnittdarstellung einer weiteren, modifizierten Ausführungsform der Erfindung; und
Fig. 10 eine Axialschnittdarstellung einer weiteren, modifizierten Ausführungsform der Erfindung.

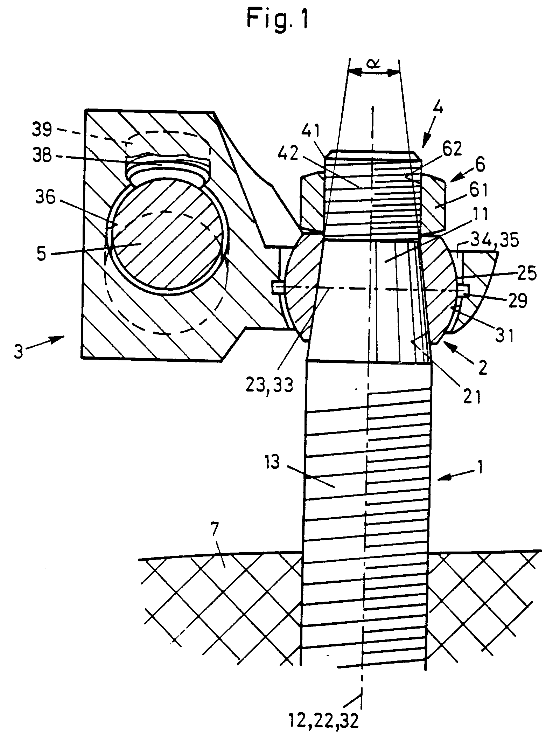

Die in Fig. 1 dargestellte Fixationsvorrichtung ist für den Einsatz des Implantates im Sakralbereich bestimmt. Sie besteht im wesentlichen aus dem hier als Pedikelschraube ausgebildeten Fixationselement 1, dem Klemmelement 2, welches im Verbindungselement 3 gelagert ist und dem Sicherungselement 6.

Die Pedikelschraube weist einen konischen Kopfteil 11 und einen daran anschliessenden, zur Befestigung im Knochen 7 bestimmten, hier als Gewindeschaft ausgebildeten Verankerungsteil 13 auf. Die Pedikelschraube weist ferner einen in ihrer Längsachse 12 angeordneten kreiszylindrischen Abschnitt 41 mit einem Aussengewinde 42 auf, welcher als Zugelement 4 dient und die axiale Verschiebung und Verkeilung des konischen Kopfteils 11 gegenüber dem Klemmelement 2 gestattet. Der konische Kopfteil 11 verjüngt sich in Richtung seines freien, vom Gewindeschaft abgewandten Endes unter einem Konuswinkel α/2 von ca. 4°.

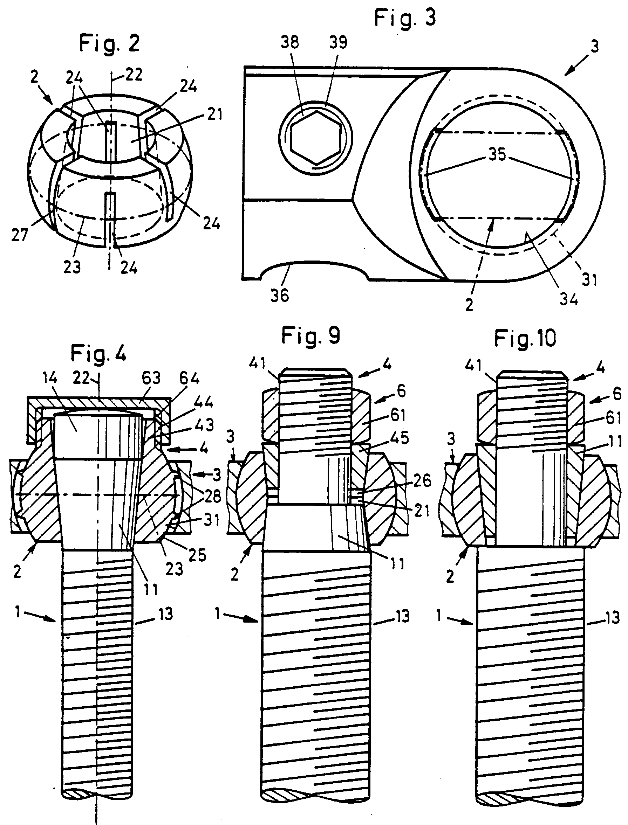

Das in Fig. 2 im Detail dargestellte Klemmelement 2 weist eine konische Bohrung 21 zur form- und kraftschlüssigen Aufnahme des konischen Kopfteils 11 der Pedikelschraube auf. Es ist kugelschichtförmig ausgebildet und erstreckt sich beidseits eines Grosskreises 23. Seine Längsachse 22 fällt in der Fig. 1 mit der Längsachse 12 der Pedikelschraube zusammen. Da die beiden Elemente 1,2 jedoch vor ihrer gegenseitigen Blockierung gegeneinander drehbar angeordnet sind, können die beiden Längsachsen 12, 22 innerhalb eines weiten Bereiches voneinander abweichen. Das Klemmelement 2 ist mit quer zum Grosskreis 23 verlaufenden Schlitzen 24 versehen, welche alternierend einmal von oben und einmal von unten angeordnet sind. Einer dieser Schlitze 27 ist durchgehend ausgebildet. Bei einer zeichnerisch nicht dargestellten Variante können die Schlitze 24 auch nur von der Seite des grösseren Durchmessers der konischen Bohrung 21 bis zum Grosskreis 23 hin geführt werden.

Das bei dieser Ausführungsform als Sakralbacke ausgebildete Verbindungselement 3 weist eine kugelschichtförmige Bohrung 31 zur formschlüssigen Aufnahme des kugelschichtförmigen Klemmelementes 2 auf. Die Längsachse 32 der Bohrung 31 fällt in Fig. 1 mit den Achsen 12,22 zusammen. Die Oberfläche der kugelschichtförmigen Bohrung 31 erstreckt sich beidseits eines Grosskreises 33, so dass das Klemmelement 2 darin sicher eingebettet ist und ein Herausstossen in axialer Richtung 12,22,32 von unten oder von oben ausgeschlossen ist. Die kugelschichtförmige Bohrung 31 weist ferner eine scharfkantige Nut 29 auf; dies ergibt eine verbesserte Verklemmung insbesondere, wenn das Klemmelement 2 aus einem weicheren Material besteht als Fixationselement 1.

Wie in Fig. 3 dargestellt ist die kugelschichtförmige Bohrung 31 des als Sakralbacke ausgebildeten Verbindungselementes 3 an seiner unteren Öffnungen 34 mit zwei um 180° versetzt angeordneten Ausnehmungen 35 versehen, welche das Einsetzen und Entfernen des Klemmelementes 2 gestatten. Zu diesem Zweck wird das Klemmelement 2 um 90° gedreht, so dass seine Längsachse 22 senkrecht zur Längsachse 32 der Bohrung 31 steht, und seine Berührungsoberfläche zu den Ausnehmungen 35 ausgerichtet ist. Das Klemmelement 2 kann dann ohne Kraftanwendung der Bohrung 31 entnommen werden.

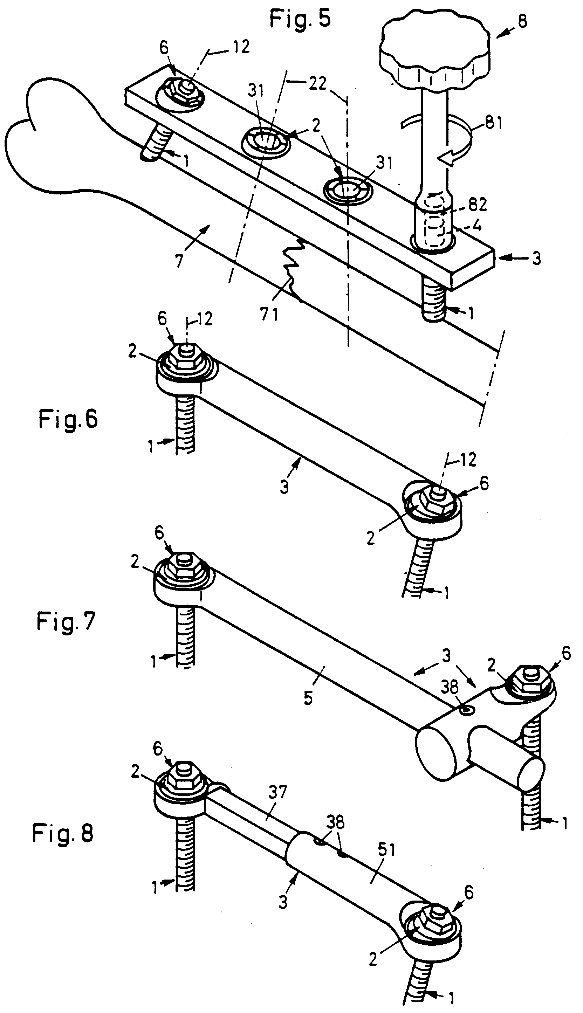

Die Blockierung der erfindungsgemässen Fixationsvorrichtung erfolgt durch Anwendung eines in Fig. 5 dargestellten Instrumentes 8, das an seinem vorderen Ende ein Innengewinde 82 aufweist. Durch Drehen im Uhrzeigersinn wird das Aussengewinde 42 des Zugelementes 4 so lange in das Innengewinde 82 eingeschraubt bis das Instrument 8 am Klemmelement 2 anstösst und dadurch das Fixationselement 1 axial in die konische Bohrung 21 hineinzieht, wodurch sich das Klemmelement 2 dank seiner Schlitze 24 aufweitet und in der Bohrung 31 verklemmt wird. Die Oberfläche 25 des kugelzonenförmigen Klemmelementes 2 und/oder die kugelige Innenfläche des Verbindungselementes 3 ist zweckmässigerweise aufgerauht oder strukturiert, so dass eine optimale Verklemmung in der Bohrung 31 erreicht wird.
Gleichzeitig werden natürlich auch die beiden Elemente 1,2 längs ihrer konischen Flächen gegeneinander verklemmt. Die Konuswinkel α/2, d.h. des konischen Kopfteils 11 und der konischen Bohrung 21, betragen beide 4°. Bei einem Konuswinkel α/2 dieser Grösse erfolgt eine optimale Selbsthemmung der Fixation der beiden Elemente 1 und 2 gegeneinander.

Das als Sakralbacke ausgebildete Verbindungselement 3 ist weiter mit einem kreiszylindrischen Kanal 36 (Fig. 1) versehen, der bezüglich der Horizontalebene (wie sie durch den Grosskreis 33 definiert ist) einen Winkel von 25° einschliesst. Im Kanal 36 ist ein Längsträger 5 eingeführt, der mittels der Stellschraube 38 in der zum Kanal 36 führenden Bohrung 39 in jeder beliebigen Lage fixiert werden kann.

Nach erfolgter Blockierung der einzelnen Elemente wird eine als Sicherungselement 6 wirkende Mutter 61 mit einem zum Aussengewinde 42 des Zugelementes 4 korrespondierenden Innengewinde 62 auf den kreiszylindrischen Abschnitt 43 aufgeschraubt.

In Fig. 4 ist eine Variante der erfindungsgemässen Fixationsvorrichtung dargestellt, bei der das Zugelement 4 nicht am Fixationselement 1, sondern axial am Klemmteil 2 angebracht ist. Das Zugelement 4 besteht hier aus einem mit der Längsachse 22 des Klemmelementes 2 fluchtenden, kreiszylindrischen Abschnitt 43 mit Aussengewinde 44. Der konische Kopfteil 11 des Fixationselementes 1 besitzt hier eine kreiszylindrische Fortsetzung 14.
Die gegenseitige Blockierung der Elemente 1,2 kann mittels des gleichen Instrumentes 8 (Fig. 5), allerdings mit einem relativ kurzen Innengewinde 82 erfolgen. Durch Drehung des Instrumentes 8 im Uhrzeigersinn wird das Aussengewinde 44 wiederum in das Innengewinde 82 hineingedreht bis das Instrument 8 an die Fortsetzung 14 stösst und damit die gleichen Vorgänge auslöst wie bei der Ausführungsform gemäss den Fig. 1 - 3.
Zwecks Verbesserung der Fixation weist die kugelzonenförmige Oberfläche 25 des Klemmelementes 2 zum Grosskreis 23 parallel verlaufende scharfe Kanten 28 auf und das Verbindungselement 3 ist bei dieser Ausführungsform aus einem weicheren Material gefertigt als das Klemmelement 2.
Nach erfolgter Blockierung der einzelnen Elemente wird eine als Sicherungselement 6 wirkende Kappe 63 mit einem zum Aussengewinde 44 korrespondierenden Innengewinde 64 auf den kreiszylindrischen Abschnitt 43 aufgeschraubt.

Wie in Fig. 5 dargestellt kann das Verbindungselement 3 der erfindungsgemässen Fixationsvorrichtung auch als Knochenplatte ausgebildet werden. In die vier kugelschichtförmigen Bohrungen 31 sind vier Klemmelemente 2 eingepasst in welche je nach Bedarf Knochenschrauben in Form der Fixationselemente 1 nach Fig. 1 befestigt werden können.

In Fig. 6 ist eine erfindungsgemässe Fixationsvorrichtung in Form eines doppelseitigen Fixateur interne mit fester Länge dargestellt. Die beiden Enden des Verbindungselementes 3 weisen je eine kugelschichtförmige Bohrung 31 mit eingepasstem Klemmelement 2 auf, in welche die Fixationselemente 1 eingebracht und darin verklemmt werden können. Zur Sicherung der Fixation sind Muttern 6 vorgesehen. Die Lage der Längsachsen 12 der beiden Fixationselemente 1 ist dank der kugeligen Klemmelemente 2 innerhalb eines weiten Winkelbereiches einstellbar.

In Fig. 7 ist eine erfindungsgemässe Fixationsvorrichtung in Form eines einseitigen Fixateur interne dargestellt, der aus einem Längsträger 5 mit einer einzelnen an seinem linken Ende angebrachten kugelschichtförmige Bohrung 31 mit eingepasstem Klemmelement 2 besteht. Am rechten Ende des Längsträgers 5 ist ein zur Sakralbacke gemäss Fig. 1 analoges Verbindungselement 3 auf den Längsträger 5 aufgeschoben und mittels der Stellschraube 38 daran lösbar fixiert, so dass es beliebig hin und her verschoben werden kann. Zur Sicherung der Fixation sind wiederum Muttern 6 vorgesehen.

In Fig. 8 ist eine erfindungsgemässe Fixationsvorrichtung in Form eines universellen, längsverstellbaren Fixateur interne dargestellt. Er besteht aus einem zur Sakralbacke gemäss Fig. 1 analogen Verbindungselement 3 mit daran anschliessender Vierkantpartie 37 und einem ebensolchen Verbindungselement 3 mit daran anschliessendem Hohlkörper 51 mit Vierkantbohrung. Die Vierkantpartie 37 kann somit längsverschieblich in den Hohlkörper 51 eingeführt und mittels der beiden Stellschrauben 38 in jeder beliebigen Lage fixiert werden. Beide Verbindungselemente 3 weisen eine kugelschichtförmige Bohrung 31 mit eingepasstem Klemmelement 2 auf, in welchen je ein Fixationselement 1 eingeführt, verklemmt und mittels der Mutter 6 gesichert werden kann.
Statt einem vierkantigen Querschnitt der beiden teleskopierenden Elemente 35 und 51 kann ein beliebiger polygonaler oder auch kreisförmiger Querschnitt gewählt werden. Bei einem kreisförmigen Querschnitt wird die Oberfläche der beiden Elemente 35 und 51 in ihrem Berührungsbereich vorteilhafterweise längsverzahnt, um eine rotationsstabile Verbindung zu erhalten.

Fig. 9 zeigt eine weitere Variante der erfindungsgemässen Fixationsvorrichtung, bei welcher der konische Kopfteil 11 des als Knochenschraube ausgebildeten Fixationselementes 1 relativ kurz bemessen ist. Gleichermassen erstreckt sich die sich von unten her verjüngende, konische Bohrung 21 des Klemmelementes 2 nur auf eine beschränkte Höhe desselben, um sich dann nach oben als obere konische Bohrung 26 zu erweitern. Die Montage dieser Fixationsvorrichtung erfolgt im wesentlichen identisch zur Ausführung gemäss den Fig. 1 - 3, bloss dass ein zur oberen konischen Bohrung 26 korrespondierender Hohlkegel 45 über den kreiszylindrischen Abschnitt 41 des Zugelementes 4 geschoben wird, der sich beim axialen Verschieben der beiden Elemente 1,2 in analoger Weise verklemmt.

Fig. 10 zeigt schliesslich eine Variante der erfindungsgemässen Fixationsvorrichtung, bei welcher der konische Kopfteil 11 des als Knochenschraube ausgebildeten Fixationselementes 1 nicht fest mit dem Verankerungsteil 13 verbunden ist, sondern als separater Hohlkegel ausgebildet ist, welcher auf den kreiszylindrischen Abschnitt 41 des Zugelementes 4 aufgeschoben werden kann. Der Verklemmechanismus bleibt gegenüber den oben beschriebenen Ausführungsformen identisch.

## Patentansprüche

1. Osteosynthetische Fixationsvorrichtung mit
A) einem Fixationselement (1) mit Längsachse (12), welches einen mindestens teilweise konischen Kopfteil (11) und einen daran anstossenden, zur Befestigung im oder am Knochen bestimmten Verankerungsteil (13) aufweist; und
B) einem, eine konische Bohrung (21) zur form- oder kraftschlüssigen Aufnahme des konischen Kopfteils (11) aufweisenden, sich beidseits eines Grosskreises (23) erstreckenden, kugelschichtförmigen Klemmelement (2) mit Längsachse (22), das mit quer zum Grosskreis (23) verlaufenden Schlitzen (24) versehen ist und zur Verklemmung innerhalb eines mit einer Bohrung (31) mit kugelzonenförmiger Innenfläche ausgestatteten Verbindungselementes (3) bestimmt ist;
dadurch gekennzeichnet, dass
C) das Fixationselement (1) oder das Klemmelement (2) mit einem axial angeordneten Zugelement (4) versehen ist, welches eine axiale Verschiebung und Verkeilung des konischen Kopfteils (11) in der damit korrespondierenden Bohrung (21) gestattet; und
D) das Zugelement (4) in Richtung der Längsachse (12) des Fixationselementes (1) gesehen auf der dem Verankerungsteil (13) abgewandten Seite des konischen Kopfteils (11) angeordnet ist.

2. Fixationsvorrichtung nach Anspruch 1, gekennzeichnet durch ein Verbindungselement (3), welches eine eine kugelzonenförmige Innenfläche aufweisende Bohrung (31) mit Längsachse (32) aufweist, welche der formschlüssigen Aufnahme des kugelschichtförmigen Klemmelementes (2) dient.

3. Fixationsvorrichtung nach Anspruch 2, dadurch gekennzeichnet, dass die Oberfläche der kugelzonenförmigen Innenfläche der Bohrung (31) des Verbindungselementes (3) sich beidseits eines Grosskreises (33) erstreckt.

4. Fixationsvorrichtung nach Anspruch 2 oder 3, dadurch gekennzeichnet, dass das Klemmelement (2) drehbar, jedoch nicht entfernbar innerhalb der kugelzonenförmigen Innenfläche der Bohrung (31) des Verbindungselementes (3) gelagert ist.

5. Fixationsvorrichtung nach Anspruch 2 oder 3, dadurch gekennzeichnet, dass die kugelzonenförmige Innenfläche der Bohrung (31) des Verbindungselementes (3) an einer ihrer beiden Öffnungen (34) mit zwei um 180° versetzt angeordneten Ausnehmungen (35) versehen ist, welche das Einsetzen und Entfernen des Klemmelementes (2) gestatten.

6. Fixationsvorrichtung nach einem der Ansprüche 1 - 5, dadurch gekennzeichnet, dass sich der konische Kopfteil (11) des Fixationselementes (1) in Richtung seines freien, vom Verankerungsteil (12) abgewandten Endes verjüngt.

7. Fixationsvorrichtung nach einem der Ansprüche 1 - 6, dadurch gekennzeichnet, dass der Konuswinkel α/2 des konischen Kopfteils (11) und der konischen Bohrung (21) im Bereich von 2° - 7°, vorzugsweise von 3° - 5° liegt.

8. Fixationsvorrichtung nach einem der Ansprüche 1 - 7, dadurch gekennzeichnet, dass die quer zum Grosskreis (23) des Klemmelementes (3) verlaufenden Schlitze (24) alternierend einmal von oben und einmal von unten angeordnet sind.

9. Fixationsvorrichtung nach einem der Ansprüche 1 - 8, dadurch gekennzeichnet, dass einer der quer zum Grosskreis (23) des Klemmelementes (3) verlaufenden Schlitze (24) von oben nach unten durchgehend ist.

10. Fixationsvorrichtung nach einem der Ansprüche 2 - 9, dadurch gekennzeichnet, dass die kugelzonenförmige Oberfläche (25) des Klemmelementes (2) und/oder die kugelzonenförmige Innenfläche der Bohrung (31) des Verbindungselementes (3) aufgerauht ist.

11. Fixationsvorrichtung nach einem der Ansprüche 2 - 10, dadurch gekennzeichnet, dass die kugelzonenförmige Innenfläche der Bohrung (31) strukturiert ist, vorzugsweise in Form einer scharfkantigen Nut (29), und das Klemmelement (2) aus einem weicheren Material besteht als das Fixationselement (1).

12. Fixationsvorrichtung nach einem der Ansprüche 1 - 11, dadurch gekennzeichnet, dass die kugelzonenförmige Oberfläche (25) des Klemmelementes (2) strukturiert ist, vorzugsweise in Form von hervorstehenden, scharfen Kanten und das Verbindungselement (3) aus einem weicheren Material besteht als das Klemmelement (2).

13. Fixationsvorrichtung nach einem der Ansprüche 2 - 12, dadurch gekennzeichnet, dass das Verbindungselement (3) mit einem, vorzugsweise kreiszylindrischen Kanal (36) versehen ist zur Aufnahme eines Längsträgers (5).

14. Fixationsvorrichtung nach einem der Ansprüche 1 - 13, dadurch gekennzeichnet, dass das axial angeordnete Zugelement (4) ein axial mit dem Kopfteil (11) fluchtender kreiszylindrischer Abschnitt (41) mit Aussengewinde (42) ist.

15. Fixationsvorrichtung nach einem der Ansprüche 1 - 13, dadurch gekennzeichnet, dass das axial angeordnete Zugelement (4) ein axial mit dem Klemmteil (2) fluchtender kreiszylindrischer Abschnitt (43) mit Aussengewinde (44) ist.

16. Fixationsvorrichtung nach Anspruch 14, dadurch gekennzeichnet, dass zusätzlich ein Sicherungselement (6) vorgesehen ist, vorzugsweise eine Mutter (61) mit einem zum Aussengewinde (42) korrespondierenden Innengewinde (62).

17. Fixationsvorrichtung nach Anspruch 15, dadurch gekennzeichnet, dass zusätzlich ein Sicherungselement (6) vorgesehen ist, vorzugsweise eine Kappe (63) mit einem zum Aussengewinde (44) korrespondierenden Innengewinde (64).

18. Fixationsvorrichtung nach einem der Ansprüche 2 - 17, gekennzeichnet durch N Fixationselemente (1), welche mittels N Klemmelementen (2) in N Bohrungen (31) mit kugelzonenförmigen Innenflächen eines einzigen Verbindungselementes (3) befestigbar sind.

19. Fixationsvorrichtung nach einem der Ansprüche 1 - 18, dadurch gekennzeichnet, dass der konischen Kopfteil (11) und der Verankerungsteil (13) einstückig ausgebildet sind.

20. Fixationsvorrichtung nach einem der Ansprüche 1 - 18, dadurch gekennzeichnet, dass der konischen Kopfteil (11) und der Verankerungsteil (13) zweistückig ausgebildet sind, wobei der Kopfteil (11) vorzugsweise als Hohlkegel ausgebildet ist.

21. Fixationsvorrichtung nach einem der Ansprüche 2 - 20, gekennzeichnet durch N Fixationselemente (1), welche mittels N Klemmelementen (2) in N Bohrungen (31) mit kugelzonenförmigen Innenflächen zweier miteinander verbindbarer Verbindungselemente (3) befestigbar sind.

22. Fixationsvorrichtung nach einem der Ansprüche 1 - 7 oder 10 - 12, dadurch gekennzeichnet, dass die quer zum Grosskreis (23) des Klemmelementes (2) verlaufenden Schlitze (24) nur von der Seite des grösseren Durchmessers der konischen Bohrung (21) bis zum Grosskreis (23) hin geführt sind.

23. Fixationsvorrichtung nach Anspruch 22, dadurch gekennzeichnet, dass einer der quer zum Grosskreis (23) des Klemmelementes (2) verlaufenden Schlitze (24) von unten nach oben durchgehend ist.

## Claims

1. An osteosynthetic fixation device with
A) a fixation piece (1) with longitudinal axis (12) which has a head section (11) which is at least partially conical and an anchoring piece (13) abutting it for attachment into or onto bone; and
B) a spherical-layer-forming clamping piece (2) with a longitudinal axis (22), said clamping piece (2) extending on both sides of a great circle (23) and being provided with a conical borehole (21) for form-locking and force-locking installation of said conical head section (11), said clamping piece (2) being provided with slits (24) running transverse to great circle (23), said clamping piece (2) being destined for locking within a connecting piece (3) which is equipped with a borehole (31) having a spherical-zone-shaped interior surface;
**characterized in that**
C) said fixation piece (1) or the clamping piece (2) is provided with an axially arrayed tension piece (4), which permits axial displacement and wedging of conical head section (11) in borehole (21) that corresponds with it; and
D) said tension piece (4) is arranged on the side opposite to said anchoring piece (13) of said conical head section (11) seen in direction of said longitudinal axis (12).

2. Fixation device according to claim 1, characterized by a connecting piece (3) having a borehole (31) with a spherical-zone-shaped interior surface with longitudinal axis (32), said connecting piece (3) being destined for form-locking installation of spherical-layer-forming clamping piece (2).

3. Fixation device according to claim 2, characterized in that the surface of the spherical-zone-shaped interior surface of said borehole (31) of connecting element (3) extends on both sides of great circle (33).

4. Fixation device according to claim 2 or 3, characterized in that clamping piece (2) is seated within the spherical-zone-shaped interior surface of said borehole (31) of connecting piece (3) in such a way as to be turnable, but not removable.

5. Fixation device according to claim 2 or 3, characterized in that spherical-zone-shaped interior surface of said borehole (31) of connecting piece (3) is provided at one of its two openings (34) with two recesses (35) arrayed so as to be offset by 180°, permitting insertion and removal of clamping piece (2).

6. Fixation device according to one of the claims 1 to 5, characterized in that conical head section (11) of fixation piece (1) tapers down in the direction of its free end which is turned away from the anchoring piece (12).

7. Fixation device according to one of the claims 1 to 6, characterized in that the conical angle α/2 of conical head section (11) and conical borehole (21) is in the range of 2°-7°, preferably in the range of 3°- 5°.

8. Fixation device according to one of the claims 1 to 7, characterized in that the slits (24) which run transverse to great circle (23) of clamping piece (3) are arranged so as to extend in alternately from above and below.

9. Fixation device according to one of the claims 1 to 8, characterized in that one of the slits (24) running transverse to great circle (23) of clamping piece (3) runs continuously through from top to bottom.

10. Fixation device according to one of the claims 2 to 9, characterized in that the spherical-zone-shaped surface (25) of clamping piece (2) and/or spherical-zone-shaped interior surface of borehole (31) of connecting piece (3) is/are made rough.

11. Fixation device according to one of the claims 2 to 10, characterized in that spherical-zone-shaped interior surface of borehole (31) is structurally designed preferably in the form of a sharp-edged groove (29), and clamping piece (2) consists of a softer material than fixation piece (1).

12. Fixation device according to one of the claims 1 to 11, characterized in that spherical-zone-forming surface (25) of clamping piece (2) is structurally designed, preferably in the form of overhanging sharp edges, and connecting piece (3) consists of a softer material than clamping piece (2).

13. Fixation device according to one of the claims 2 to 12, characterized in that connecting piece (3) is provided with a channel (36), preferably circular cylindrical, for installation of a longitudinal outrigger (5).

14. Fixation device according to one of the claims 1 to 13, characterized in that axially arrayed tension element (4) is a circular cylindrical segment (41) aligned with head section (11) and is equipped with an exterior threading (42).

15. Fixation device according to one of the claims 1 to 13, characterized in that axially arrayed tension element (4) is a circular cylindrical segment (43) aligned with the clamping piece (2) and is equipped with an exterior threading (44).

16. Fixation device according to claim 14, characterized in that additionally a fastening piece (6) is provided, preferably a nut (61) with an interior threading (62) corresponding to exterior threading (42).

17. Fixation device according to claim 15, characterized in that additionally a fastening piece (6) is provided, preferably a cap (63) with an interior threading (64) corresponding to exterior threading (44).

18. Fixation device according to one of the claims 2 to 17, characterized by N fixation piece (1), which are attachable by means of N clamping pieces (2) into N boreholes (31) with spherical-zone-shaped interior surfaces of a single connecting piece (3).

19. Fixation device according to one of the claims 1 to 18, characterized in that conical head section (11) and anchoring piece (13) are formed as a single piece.

20. Fixation device according to one of the claims 1 to 18, characterized in that conical head section (11) and anchoring piece (13) are formed as two pieces, with head section (11) preferably formed as a hollow cone.

21. Fixation device according to one of the claims 2 to 20, characterized by N fixation pieces (1) which are attachable by means of N clamping pieces (2) into N boreholes (31) with spherical-zone-shaped interior surfaces of two connecting pieces (3) which can be joined to each other.

22. Fixation device according to one of the claims 1 to 7 or 10 to 12, characterized in that the slits (24) running transverse to great circle (23) of clamping piece (2) run in only from the side of the larger diameter of conical borehole (21) to great circle (23).

23. Fixation device according to claim 22, characterized in that one of the slits (24) running transverse to great circle (23) of clamping piece (2) runs continuously through from bottom to top.

## Revendications

1. Dispositif d'immobilisation pour ostéosynthèse, comportant:
A) un élément d'immobilisation (1) présentant un axe longitudinal (12), et qui présente une pièce de tête (11) au moins partiellement conique et une pièce d'ancrage (13) qui vient buter contre elle, et destinée à la fixation dans l'os ou sur l'os; et
B) un élément de serrage (2) présentant un axe longitudinal (22), en forme de tranche de sphère, s'étendant des deux côtés d'un grand cercle (23), et présentant un alésage conique (21) en vue de la réception en correspondance géométrique et à serrage mécanique de la pièce conique de tête (11), qui est doté de fentes (24) s'étendant transversalement par rapport au grand cercle (23) et qui est destiné à être serré à l'intérieur d'un élément de liaison (3) doté d'un alésage (31) présentant une surface intérieure en forme de tranche de sphère;
caractérisé en ce que
C) l'élément d'immobilisation (1) ou l'élément de serrage (2) sont dotés d'un élément de traction (4) disposé axialement, qui permet un coulissement et un coinçage axiaux de la pièce de tête (11) conique dans l'alésage (21) qui lui correspond; et
D) l'élément de traction (4), vu dans la direction de l'axe longitudinal (12) de l'élément d'immobilisation (1), est disposé sur le côté de la pièce de tête (11) conique qui n'est pas tourné vers la pièce d'ancrage (13).

2. Dispositif d'immobilisation selon la revendication 1, caractérisé par un élément de liaison (3) qui présente un alésage (31) dont la surface intérieure est en forme de tranche de sphère et avec un axe longitudinal (32), et qui sert à la réception en correspondance géométrique de l'élément de serrage (2) en forme de tranche de sphère.

3. Dispositif d'immobilisation selon la revendication 2, caractérisé en ce que la surface intérieure en forme de tranche de sphère de l'alésage (31) de l'élément de liaison (3) s'étend des deux côtés d'un grand cercle (33).

4. Dispositif d'immobilisation selon la revendication 2 ou 3, caractérisé en ce que l'élément de serrage (2) est monté de manière à pouvoir tourner, mais sans pouvoir être enlevé, à l'intérieur de la surface intérieure en forme de tranche de sphère de l'alésage (31) de l'élément de liaison (3).

5. Dispositif d'immobilisation selon la revendication 2 ou 3, caractérisé en ce que la surface intérieure en forme de tranche de sphère de l'alésage (31) de l'élément de liaison (3) est doté à l'une de ses deux ouvertures (34) de deux évidements (35) décalés de 180° et qui permettent l'insertion et l'enlèvement de l'élément de serrage (2).

6. Dispositif d'immobilisation selon l'une des revendications 1 à 5, caractérisé en ce que la pièce de tête (11) conique de l'élément d'immobilisation (1) se rétrécit en direction de son extrémité libre qui n'est pas tournée vers la pièce d'ancrage (12).

7. Dispositif d'immobilisation selon l'une des revendications 1 à 6, caractérisé en ce que l'angle de cône α/2 de la pièce de tête (11) conique et de l'alésage conique (21) est situé dans l'intervalle allant de 2° à 7°, et de préférence de 3° à 5°.

8. Dispositif d'immobilisation selon l'une des revendications 1 à 7, caractérisé en ce que les fentes (24) qui s'étendent transversalement par rapport au grand cercle (23) de l'élément de serrage (3) sont disposées en alternance, l'une partant du haut et l'autre partant du bas.

9. Dispositif d'immobilisation selon l'une des revendications 1 à 8, caractérisé en ce qu'une des fentes (24) s'étendant transversalement par rapport au grand cercle (23) de l'élément de serrage (3) s'étend sans discontinuer du haut en bas.

10. Dispositif d'immobilisation selon l'une des revendications 2 à 9, caractérisé en ce que la surface (25) en forme de tranche de sphère de l'élément de serrage (2) et/ou la surface intérieure en forme de tranche de sphère de l'alésage (31) de l'élément de liaison (3) sont rendues rugueuses.

11. Dispositif d'immobilisation selon l'une des revendications 2 à 10, caractérisé en ce que la surface intérieure en forme de tranche de sphère de l'alésage (31) est structurée, de préférence sous la forme d'une rainure (29) à bords aigus, et l'élément de serrage (2) est réalisé en un matériau moins dur que l'élément d'immobilisation (1).

12. Dispositif d'immobilisation selon l'une des revendications 1 à 11, caractérisé en ce que la surface (25) en forme de tranche de sphère de l'élément de serrage (2) est structurée, de préférence sous la forme d'arêtes aiguës en saillie, et en ce que l'élément de liaison (3) est réalisé en un matériau moins dur que l'élément de serrage (2).

13. Dispositif d'immobilisation selon l'une des revendications 2 à 12, caractérisé en ce que l'élément de liaison (3) est doté d'un canal (36) de préférence cylindrique circulaire, pour la réception d'un support longitudinal (5).

14. Dispositif d'immobilisation selon l'une des revendications 1 à 13, caractérisé en ce que l'élément de traction (4) disposé axialement est un tronçon cylindrique circulaire (41) à filet extérieur (42), aligné axialement sur la pièce de tête (11).

15. Dispositif d'immobilisation selon l'une des revendications 1 à 13, caractérisé en ce que l'élément de traction (4) disposé axialement est un tronçon cylindrique circulaire (43) à filet extérieur (44), aligné axialement sur la pièce de serrage (2).

16. Dispositif d'immobilisation selon la revendication 14, caractérisé en ce qu'il est en plus prévu un élément de protection (6), de préférence un écrou (61) présentant un filet intérieur (62) correspondant au filet extérieur (42).

17. Dispositif d'immobilisation selon la revendication 15, caractérisé en ce qu'il est en plus prévu un élément de protection (6), de préférence un bonnet (63) présentant un filet intérieur (64) correspondant au filet extérieur (44).

18. Dispositif d'immobilisation selon l'une des revendications 2 à 17, caractérisé par N éléments d'immobilisation (1), qui peuvent être fixés au moyen de N éléments de serrage (2) dans N alésages (31), présentant une surface intérieure en forme de tranche de sphère, d'un unique élément de liaison (3).

19. Dispositif d'immobilisation selon l'une des revendications 1 à 18, caractérisé en ce que la pièce de tête (11) conique et la pièce d'ancrage (13) sont réalisées en une seule pièce.

20. Dispositif d'immobilisation selon l'une des revendications 1 à 18, caractérisé en ce que la pièce de tête (11) conique et la pièce d'ancrage (13) sont configurées en deux pièces, la pièce de tête (11) étant de préférence configurée comme cône creux.

21. Dispositif d'immobilisation selon l'une des revendications 2 à 20, caractérisé par N éléments d'immobilisation (1) qui peuvent être fixés au moyen de N éléments de serrage (2) dans N alésages (31), présentant une surface intérieure en forme de tranche de sphère, de deux éléments de liaison (3) qui peuvent être reliés l'un à l'autre.

22. Dispositif d'immobilisation selon l'une des revendications 1 à 7 ou 10 à 12, caractérisé en ce que les fentes (24) s'étendant transversalement par rapport au grand cercle (23) de l'élément de serrage (2) ne s'étendent que depuis le côté de plus grand diamètre de l'alésage conique (21) jusqu'au grand cercle (23).

23. Dispositif d'immobilisation selon la revendication 22, caractérisé en ce que l'une des fentes (24) s'étendant transversalement par rapport au grand cercle (23) de l'élément de serrage (2) s'étend sans discontinuer du bas vers le haut.1
